## Europäisches Patentamt

## European Patent Office

## Office européen des brevets

(19)

(11) Publication number: **0 138 551**
A2

(12) # EUROPEAN PATENT APPLICATION

(21) Application number: **84306865.1**

(22) Date of filing: **09.10.84**

(51) Int. Cl.⁴: **A 61 L 15/03**, A 61 L 15/06,
A 61 M 37/00

(30) Priority: **11.10.83 US 540249**

(43) Date of publication of application: **24.04.85**
**Bulletin 85/17**

(84) Designated Contracting States: **BE DE FR GB IT NL**

(71) Applicant: **WARNER-LAMBERT COMPANY, 201 Tabor
Road, Morris Plains New Jersey 07950 (US)**

(72) Inventor: **Belsole, Susan C., RD No. 1, Box 207C, South
Road, Chester New Jersey 07930 (US)**

(74) Representative: **Jones, Michael Raymond et al,
HASELTINE LAKE & CO. Hazlitt House 28 Southampton
Buildings Chancery Lane, London WC2A 1AT (GB)**

(54) **A system for the transdermal delivery of nitroglycerin.**

(57) There is disclosed a tape for the transdermal delivery of
nitroglycerin for the treatment of heart disease comprising, in
one form,

a substantially impervious foil or film;

coated on a portion of one side of the foil or film, a nitroglycerin-containing layer which comprises a polymeric material
and the nitroglycerin; and

adhesive means over at least a different portion of the one
side of the foil or film not coated by the nitroglycerin-containing
layer for securing the tape to the skin of a patient, whilst permitting said layer to contact the skin.

Another form of a tape comprises

a substantially impervious foil or film;

coated on one side of the foil or film, a nitroglycerin-containing layer which comprises a polymeric material and the
nitroglycerin; and

provided on the opposite, uncoated, side of the foil or film,
and adhesive strip which projects beyond the periphery of the
foil or film, for securing the tape to the skin of a patient with
said layer in contact with the skin.

ACTORUM AG

-1-

# A SYSTEM FOR THE TRANSDERMAL DELIVERY OF NITROGLYCERIN

This invention relates to a system for the transdermal delivery of nitroglycerin and is more particularly, although not exclusively, concerned with a simple nitroglycerin-bearing tape which is conveniently applied to the skin.

It is known that nitroglycerin is pharmaceutically useful in the treatment or prevention of angina pectoris, myocardial infarction paroxysm, and cardiac insufficiency.

Among internal treatments for ischemic heart disease is vasodilator therapy in which nitroglycerin is used as a coronary vasodilator. Its effects include dilation of the coronary artery, releasing contraction of the coronary artery and dilation of collateral and resistance vessels thereby increasing the oxygen supply to the ischemic area of cardiac muscle. It is also known that nitroglycerin has effects such as dilation of arteries and reduction of blood pressure throughout the body (thereby reducing the load applied to the heart), dilation of veins and reduction of the amount of venous return, thereby reducing the forward-load to the heart and reducing the oxygen consumption in the cardiac muscle.

Conventionally, nitroglycerin has been administered by intravenous injection, as a sublingual tablet or as an ointment.

Sublingual tablets are generally used for the treatment of paroxysm due to angina pectoris but the duration of effectiveness is rather short, for instance 20 to 30 minutes.

When prolonged effectiveness is required, for instance in the treatment of cardiac insufficiency or in the prevention of angina pectoris, nitroglycerin has generally been administered as an ointment. The duration of effectiveness of the ointment application may be up to four to eight hours.

The ointment is generally applied using a scaled parchment paper applicator which may be for instance 6 cm x 9 cm in size. The amount of ointment applied

0138551

-3-

is generally the maximum that can be tolerated without the onset of headache, this being a symptom of undesirable side effects. For instance 0.35 g ointment (containing 7.5 mg nitroglycerin) may be applied to the frontal chest skin and further amounts of 0.35 g until headache develops which shows the amount is then too great. Generally, the amount is from 0.7 to 1.5 g (containing 14 to 30 mg nitroglycerin) and the ointment is generally applied over 200 $cm^2$ or more of the frontal chest skin.

To prevent evaporation of nitroglycerin and to prevent staining of clothes, the ointment is generally covered with a plastic film fixed to the skin by adhesive tapes.

The administration of ointment is thus rather inconvenient. It is difficult to determine the exact amount to be applied, and its application and removal can be messy.

Recently, three one-a-day type transdermal nitroglycerin (NTG) delivery systems in the form of patches were developed for controlled administration of nitroglycerin. These patches overcome the inherent problems of the ointment i.e., accuracy of dose, application, and staining. In addition, they release nitroglycerin at a uniform rate over a 24 hour period.

Belgian Patent Number 893,394 describes a membrane-controlled delivery system for NTG. Materials and manufacturing for this device are complicated and costly. US Patent Numbers 4,336,243 and 4,291,015 describe matrix-controlled delivery systems. These are both bulky and inefficient requiring a wide perimeter of adhesive to ensure intimate skin contact.

-4-

In addition, all three patches require a large excess of NTG to maintain a driving force for penetration.

Goodhart, et al., J. Pharm. Sci. __65__, No. 10, 1466 (1976) describes the stabilization of compressed NTG tablets with polyvinylpyrrolidone (PVP). It was observed that the volatility and subsequent migration of the NTG was retarded with addition of PVP. European Published Application No. 0054279 describes the use of water-insoluble PVP cross-linked or copolymerized with acrylic or vinyl esters to provide a hydrophobic matrix system for controlled release of NTG. This matrix also reduces the drug's volatility.

-5-

According to a first aspect of the present invention, there is provided a tape, suitable for use in the transdermal delivery of nitroglycerin, comprising:

a substantially impervious foil or film;

coated on a portion of one side of the foil or film, a nitroglycerin containing layer which comprises a polymeric material and the nitroglycerin; and

adhesive means over at least a different portion of the one side of the foil or film not coated by the nitroglycerin-containing layer for securing the tape to the skin of a patient, whilst permitting said layer to contact the skin.

According to a second aspect of the present invention there is provided a tape, suitable for use in the transdermal delivery of nitroglycerin, comprising:

a substantially impervious foil or film;

coated on one side of the foil or film, a nitroglycerin-containing layer which comprises a polymeric material and the nitroglycerin; and

provided on the opposite, uncoated, side of the foil or film, an adhesive strip which projects beyond the periphery of the foil or film, for securing the tape to the skin of a patient with said layer in contact with the skin.

The tape is no more obtrusive than a common adhesive strip such as is used to cover cuts and grazes.

The substantially impervious foil or film is conventionally formed from a metallic material, preferably aluminium. The polymeric material may be a water-soluble polyvinyl pyrrolidone, preferably one having a molecular weight of from 10000 to 360000. The tape may also contain plasticizers, and solvents for the nitroglycerin.

-6-

The foil or film is mounted on an adhesive strip and may be covered with a non-stick paper backing. The rate of release can be adjusted by appropriate choice of the polymeric material concentration.

In addition, the rate-controlling mechanism is such that higher blood levels of nitroglycerin can be expected from a surface area equivalent to commercially available products.

According to a third aspect of the present invention there is provided a nitroglycerin-containing layer, suitable for use in the transdermal delivery of nitroglycerin, which layer comprises nitroglycerin, a water-soluble polyvinylpyrrolidone and a plasticizer. The layer may be used in the tapes of the first aspect of this invention. The tapes of this invention are useful in the treatment of agnina pectoris by applying to the skin of a mammal suffering therefrom the above tape containing a nitroglycerin-containing polymer layer.

The nitroglycerin polymeric material complex functions as a layer former and also provides a mechanism for controlling the rate of transdermal delivery of NTG.

In use, the tape is applied to the body surface of a patient suffering from heart disease and specifically angina pectoris, whereby the nitroglycerin-containing layer and the adhesive means are in intimate contact with the patient's skin.

One embodiment of the tape may be made by preparing a nitroglycerin-polyvinylpyrrolidone solution, spreading said solution on an aluminium foil to form a layer, and allowing the layer to dry.

The nitroglycerin-polyvinylpyrrolidone solution may be prepared by dissolving nitroglycerin in a suitable solvent and further adding plasticizers and polyvinylpyrrolidone with stirring until complete solution is attained.

The polyvinylpyrrolidones (PVP) used in this embodiment to form the layer are water-soluble and can, but need not, range in molecular weight from 10,000 to 360,000. The preferred molecular weight is 360,000. The ratio of PVP to NTG can vary from 2:1 to 10:1 depending upon the molecular weight of the polymer chosen and the release rate desired. An increase in the PVP/ NTG ratio will generally decrease the rate of release.

Plasticizers compatible with PVP and NTG may be chosen from a list including glycerin, PEG 400 (polyethyleneglycol having a molecular weight of about 400), propylene glycol, sorbitol, water soluble lanolin oils, (for example PEG-75 lanolin oil), diethylene glycol, butylene glycol, acetylated lanolins, or a mixture thereof. Amounts of plasticizers in the layer, alone or in combination, may range from 15 -40%, preferably 22%, by weight of the layer.

Solvents for NTG may include octylhydroxystearate, octyl palmitate, isopropyl myristate, ispropyl palmitate, mineral oil, lanolin alcohol, dimethicone fluid or capric/caprylic triglycerides. Solvent concentration in the layer ranges from 5-25% by weight of the layer, depending on the amount of NTG present.

The plasticizer chosen may also act as a solvent for NTG as, for instance, acetylated lanolin, propylene glycol or water-soluble lanolin. When lanolin derivatives are used, the absorption of NTG will be improved. While the rate of release is controlled mainly by the choice of PVP concentration, the solvents and plasticizers used will have some effect. A product of this type, because of its simplicity, is relatively easy to manufacture and assemble.

The tape of the present invention can vary in size depending upon the dosage requirements of the patient. The NTG content of the layer may range from 10 mg to 30 mg but the preferred size is 2.5 cm x 4 cm containing 22.28 mg NTG.

The following Examples are illustrative of the invention.

-9-

## EXAMPLE 1

Preparation of nitroglycerin polyvinylpyrrolidone Solution:   A 9.1% w/w solution of NTG in ethyl alcohol USP (26.37 g) was mixed with 58.63 ml of ethyl alcohol USP until a uniform solution was obtained.  To this solution were added 1 g of PEG-75 lanolin oil, 1 g of octyl hydroxystearate and 3 g of glycerin USP. Polyvinylpyrrolidone, molecular weight 360,000 (5 g) was then added slowly and mixed until completely dissolved.

Assembly of Tape:  Approximately 0.928 ml of the nitroglycerin polyvinylpyrrolidone solution was spread evenly on a 2.5 cm x 4.0 cm rectangle of aluminium  foil and allowed to dry at room temperature. The resulting nitroglycerin-polyvinylpyrrolidone layer had a thickness of approximately 3.0 mils.  The foil strip was then centered over the adhesive side of an adhesive strip measuring 5 cm x 6.5 cm and pressed into place.  A nonstick paper backing was applied to cover both foil strip and adhesive.  The assembled tape was packaged in heat-sealable foil pouches.

## EXAMPLE 2

A nitroglycerin polyvinylpyrrolidone solution was prepared following the method and materials of Example 1 except that 10 g of PVP, molecular weight 360,000, was added.  The layer was cast on foil, assembled and packaged as in Example 1.

## EXAMPLE 3

A nitroglycerin polyvinylpyrrolidone solution was prepared following the method and materials of Example 1 except that 15 g of PVP, molecular weight 360,000, were added.  The layer was cast on foil, assembled, and packaged as in Example 1.

## EXAMPLE 4

A nitroglycerin polyvinylpyrrolidone solution was prepared following the method and materials of Example 1 except that 3 g of PVP, molecular weight 360,000, were added. The layer was cast on foil, assembled and packaged as in Example 1.

## EXAMPLE 5

Studies to determine the topical availability of nitroglycerin from the films were carried out in vitro, using hairless mouse skin. Although in vitro permeation through hairless mouse skin is not an absolute quantitation of drug delivery through human skin, the rank order of formulations tested is usually the same. Thus, commercially available products whose in vivo performance is known may be used as standards when tested in vitro under the same conditions.

The data for the in vitro release of NTG from the layers prepared in Examples 1 and 2 are set forth in the following table. Commercial standards used were a 2% NTG ointment and a transdermal delivery system of 10 cm$^2$ (TDDS).

NTG Released (mcg/cm$^2$)*

| T (hours) | Layer Ex. 1 | Layer Ex. 2 | 2% Ointment (1 mm thick) | TDDS 10 cm$^2$ |
|---|---|---|---|---|
| 1 | 0 | 0 | 10 | 7 |
| 2 | 25 | 10 | 30 | 45 |
| 4 | 60 | 50 | 65 | 85 |
| 8 | 175 | 130 | 140 | 135 |
| 12 | 330 | 220 | 210 | 190 |
| 16 | 510 | 320 | 275 | 245 |
| 20 | 680 | 420 | 335 | 290 |
| 24 | 850 | 525 | 390 | 340 |
| 30 | 1,090 | 675 | 470 | 410 |

*mean of eight determinations

Release from the ointment was linear with $T^{1/2}$ while both the layers and the TDDS exhibit zero order release rates. Thus, after an initial stabilising period, the same amount of NTG was released in every hour of the tests.

CLAIMS:

1. A tape, suitable for use in the transdermal delivery of nitroglycerin, comprising:

a substantially impervious foil or film;

coated on a portion of one side of the foil or film, a nitroglycerin-containing layer which comprises a polymeric material and the nitroglycerin; and

adhesive means over at least a different portion of the one side of the foil or film not coated by the nitroglycerin-containing layer for securing the tape to the skin of a patient, whilst permitting said layer to contact the skin.

2. A tape, suitable for use in the transdermal delivery of nitroglycerin, comprising:

a substantially impervious foil or film;

coated on one side of the foil or film, a nitroglycerin-containing layer which comprises a polymeric material and the nitroglycerin; and

provided on the opposite, uncoated, side of the foil or film, an adhesive strip which projects beyond the periphery of the foil or film, for securing the tape to the skin of a patient with said layer in contact with the skin.

3. A tape according to Claim 1 or 2, wherein the substantially impervious foil or film is formed from a metallic material, preferably aluminium.

4. A tape according to Claim 1, 2 or 3, wherein the polymeric material is a water-soluble polyvinylpyrrolidone, preferably one having a molecular weight of from 10000 to 360000.

5. A tape according to any preceding claim, wherein said layer contains a plasticizer which is preferably chosen from glycerin, polyethyleneglycol having a molecular weight of approximately 400, propylene glycol, sorbitol, PEG-75 lanolin oil, diethylene glycol, butylene glycol, acetylated

-13-

lanolin and mixtures thereof; more preferably glycerin, PEG-75 lanolin oil, propylene glycol and mixtures thereof and most preferably PEG-75 lanolin oil, glycerin and a mixture thereof.

6. A tape according to any preceding claim, wherein said layer contains from 10 to 30 mg of nitroglycerin which is dissolved in a solvent chosen from octyl hydroxystearate, octyl palmitate, isopropyl myristate, isopropyl palmitate, mineral oil, lanolin alcohol, dimethicone fluid, capric/ caprylic triglyceride, and mixtures thereof; preferably octyl hydroxystearate, octyl palmitate, lanolin alcohol and mixtures thereof; more preferably octylhydroxystearate.

7. A tape according to Claim 6, wherein the solvent is present in an amount of from 5 to 25% by weight of the layer, the polyvinylpyrrolidone is present in an amount of from 30 to 70%, preferably 40 to 60%, by weight of the layer and the plasticizer is present in an amount of from 15 to 40%, preferably 20 to 35%, more preferably 22%, by weight of the layer.

8. A tape according to any preceding claim, wherein the nitroglycerin content of the tape is in the range of from 10 to 30 mg, preferably 22 mg, more preferably 22.28 mg.

9. A tape according to Claim 8, wherein the layer comprises, in addition to the nitroglycerin, an admixture of:

about 8 % octylhydroxystearate; and about 48% polyvinylpyrrolidone, having a molecular weight of 360000, plasticized with about 8 % PEG-75 lanolin oil and about 24% glycerin;

all percentages being by weight based on the weight of the layer.

10. A tape according to Claim 8, wherein the layer comprises, in addition to the nitroglycerin, an admixture of:

about 6% octylhydroxystearate;

about 57% polyvinylpyrrolidone, having a molecular weight of 360000, plasticized with about 6% PEG-75 lanolin oil and about 17% glycerin;

all percentages being by weight based on the weight of the layer.

11. A tape according to any preceding claim, wherein the layer has the dimensions of 2.5 cm x 4 cm.

12. A nitroglycerin-containing layer, suitable for use in the transdermal delivery of nitroglycerin, which layer comprises nitroglycerin, a water-soluble polyvinylpyrrolidone and a plasticizer.

13. A nitroglycerin-containing layer according to Claim 12, wherein there is also present a solvent for the nitroglycerin and wherein the polyvinylpyrrolidone has a molecular weight of 10,000 to 360,000.

14. A nitroglycerin-containing layer, comprising from 10 to 30 mg of nitroglycerin; a solvent chosen from octyl hydroxystearate, octyl palmitate, isopropyl myristate, isopropyl palmitate, mineral oil, lanolin alcohol, dimethicone fluid and capric/ caprylic triglyceride and mixtures thereof; a water-soluble polyvinylpyrrolidone of molecular weight 10,000 to 360,000; and a plasticizer chosen from glycerin, polyethylene glycol having a molecular weight of about 400, propylene glycol, sorbitol, PEG-75 lanolin oil, diethylene glycol, butylene glycol, acetylated lanolin and mixtures thereof.

15. A nitroglycerin-containing layer comprising nitroglycerin which is present in an amount of 22.28 mg; octyl hydroxystearate which is present in an amount of about 8% by weight of the layer; polyvinylpyrrolidone, of molecular weight 360,000, which is present in an amount of 48% by weight of the layer; and PEG-75 lanolin oil and glycerin as plasticizers which are present in amounts of 8% and 25% respectively, by weight of the layer.

16. A nitroglycerin-containing layer, comprising nitroglycerin which is present in an amount of 22.28 mg; octyl hydroxystearate which is present in an amount of about 6% by weight of the layer; polyvinylpyrrolidone, of molecular weight 360,000, which is present in an amount of 57% by weight of the layer; and PEG-75 lanolin oil and glycerin as plasticizers which are present in amounts of 6% and 17%, respectively, by weight of the layer.